(19) Europäisches Patentamt
European Patent Office
Office européen des brevets

(11) Veröffentlichungsnummer: **0 050 783**
**B1**

(12) # EUROPÄISCHE PATENTSCHRIFT

(45) Veröffentlichungstag der Patentschrift: **12.12.84**

(51) Int. Cl.³: **C 07 C 51/60**, C 07 C 63/00, B 01 J 31/24, C 08 G 63/40, C 08 G 69/32

(21) Anmeldenummer: **81108198.3**

(22) Anmeldetag: **12.10.81**

(54) **Verfahren zur Herstellung von aromatischen Carbonsäurechloriden.**

(30) Priorität: **25.10.80 DE 3040294**

(43) Veröffentlichungstag der Anmeldung: **05.05.82 Patentblatt 82/18**

(45) Bekanntmachung des Hinweises auf die Patenterteilung: **12.12.84 Patentblatt 84/50**

(84) Benannte Vertragsstaaten: **DE FR GB IT NL**

(56) Entgegenhaltungen:
**DE - A - 2 933 148**
**DE - B - 1 668 276**

(73) Patentinhaber: **BAYER AG, Konzernverwaltung RP Patentabteilung, D-5090 Leverkusen 1 Bayerwerk (DE)**

(72) Erfinder: **Rudolph, Udo, Dr., Scheiblerstrasse 101, D-4150 Krefeld (DE)**
Erfinder: **Schmidt, Manfred, Dr., Bodelschwinghstrasse 20, D-4150 Krefeld (DE)**
Erfinder: **Freitag, Dieter, Dr., Hasenheide 10, D-4150 Krefeld (DE)**
Erfinder: **Bottenbruch, Ludwig, Dr., Wöhlerstrasse 5, D-4150 Krefeld (DE)**

EP 0 050 783 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft ein Einstufenverfahren zur Herstellung von hochreinen, polykondensationsfähigen aromatischen Carbonsäurechloriden.

Die Umsetzung von aliphatischen und aromatischen Carbonsäuren mit Thionylchlorid ist ein Standardverfahren der organsichen Chemie zur Herstellung der entsprechenden Säurechloride. Allerdings werden hierbei als Reaktionsprodukte dunkelgefärbte Carbonsäurechloride in einer Reinheit von 96—99% erhalten. Aromatische Dicarbonsäuredichloride dieses geringen Reinheitsgrades können nicht direkt nach dem Zweiphasengrenzflächen-Polykondensationsverfahren zur Herstellung von hochmolekularen Polykondensaten, wie aromatischen Polyamiden oder aromatischen Polyestern eingesetzt werden. Ihr Gehalt an nicht oder nur halbseitig umgesetzten Dicarbonsäuren stört die Polykondensation, verursacht unerwünschten Kettenabbruch und ergibt Polymere mit endständigen Carboxylgruppen. Die so hergestellten aromatischen Dicarbonsäuredichloride sind durch Verunreinigung dunkel gefärbt und enthalten darüber hinaus Schwefel in elementarer und gebundener Form, der die Eigenschaften der herzustellenden Polykondensate weiter beeinträchtigt.

So ist z. B. aus der DE-B 1 668 279 bekannt, daß Acrylsäure mit Phosgen in ihr Säurechlorid überführt werden kann, wenn das Katalysatorgemisch Tributylmethylphosphoniumjodid enthält.

Bei der Herstellung von Säurechloriden mit Thionylchlorid und Dimethylformamid als Katalysator (vgl. DE-PS 1 026 750) können als Nebenprodukt erhebliche Mengen von Dimethylcarbamidsäurechlorid entstehen, die das Produkt verunreinigen oder sich im eventuell zurückgeführten überschüssigen Thionylchlorid akkumulieren.

Um farblose Carbonsäurechloride zu erhalten, müssen die Rohprodukte durch Umkristallisation oder Destillation gereinigt werden. Dies erfordert zusätzlichen Aufwand und vermindert die Ausbeute; bei der Destillation besteht die Gefahr einer spontanen Zersetzung.

Gegenstand der Erfindung ist ein Einstufenverfahren zur Herstellung von reinen aromatischen Carbonsäurechloriden durch Umsetzung aromatischer Carbonsäuren mit Thionylchlorid, das dadurch gekennzeichnet ist, daß man als Katalysatoren tert. Phosphine oder deren Reaktionsprodukte mit Thionylchlorid und/oder den herzustellenden Säurechloriden verwendet. Die so erhaltenen aromatischen Carbonsäurechloride sind praktisch farblos und enthalten neben den eingesetzten Katalysatoren maximal 0,1% Verunreinigungen, so daß sie ohne Nachreinigung zur Herstellung von farblosen, hochmolekularen Polykondensaten eingesetzt werden können.

Die erfindungsgemäß als Katalysatoren eingesetzten tert. Phosphine können nach Beendigung der Reaktion in einigen Fällen einfach durch Andestillieren des Reaktionsgemisches aus diesem entfernt werden, jedoch stören verbleibende Reste des Katalysators im erhaltenen Carbonsäurechlorid die Umsetzung von beispielsweise Dicarbonsäuredichloriden zu aromatischen Polyestern nach dem Verfahren der Zweiphasengrenzflächenreaktion nicht.

Als erfindungsgemäß wirksame Katalysatoren sind tert. Phosphine der allgemeinen Struktur I geeignet

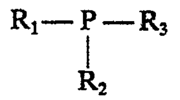

(I)

wobei $R_1$, $R_2$, $R_3$ gleich oder verschieden und $C_1-C_8$-Alkyl, $C_6-C_{10}$-Aryl, $C_7-C_{20}$-Alkylaryl oder Arylalkyl sein können. Bevorzugt sind $R_1$, $R_2$ und $R_3$ $C_6-C_{10}$-Arylreste, wie Phenyl oder mit $C_1-C_4$-Alkylresten substituiertes Phenyl.

Geeignete Katalysatoren sind beispielsweise Tribenzylphosphin, Triisopropylphosphin, Tributylphosphin und Triphenylphosphin.

Erfindungsgemäß werden 0,1 bis 5,0 Gew.-%, vorzugsweise 0,2 bis 3 Gew.-%, bezogen auf die eingesetzten aromatischen Carbonsäuren, an tert. Phosphinen der allgemeinen Struktur I eingesetzt.

Im Prinzip können alle aromatischen Carbonsäuren eingesetzt werden. Sie entsprechen insbesondere den folgenden Formeln (II) bis (VII):

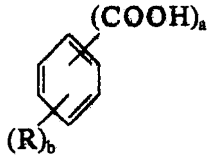

(II)

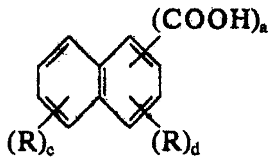

(III)

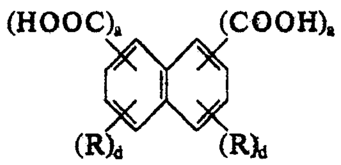

(IV)

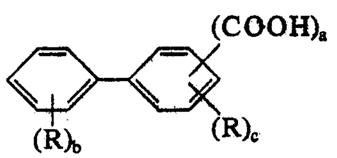

(V)

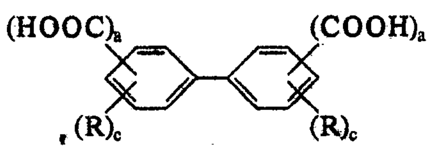

(VI)

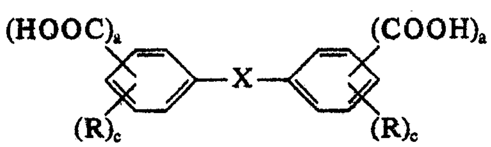

(VII)

wobei R ein Substituent aus der Gruppe von Alkylgruppen mit ein bis sechs Kohlenstoffatomen, halogensubstituierten Alkylgruppen mit ein bis sechs Kohlenstoffatomen, Alkoxygruppen und ebenfalls den entsprechenden halogensubstituierten Alkoxygruppen mit ein bis sechs Kohlenstoffatomen und Halogenatomen sein kann.

X steht für einen Ethersauerstoff, eine Methylengruppe oder Isopropylengruppe, einen $C_5-C_7$-Cycloalkylenrest oder eine $-C=O$-Gruppe, während a eine ganze Zahl von 1 bis 3, b eine ganze Zahl von 0 bis 5, c eine ganze Zahl von 0 bis 4, und d eine ganze Zahl von 0 bis 3 repräsentiert.

Beispielhaft seien genannt:

Phthalsäure, Isophthalsäure, Terephthalsäure, Gemische aus Iso- und Terephthalsäure, 4,4'-Dicarboxybenzophenon, Diphensäure, 1,4-Naphthalindicarbonsäure und Trimesinsäure.

Zur Durchführung des erfindungsgemäßen Verfahrens können die aromatischen Carbonsäuren nach Zugabe der erfindungsgemäßen Katalysatoren mit 1—2 mol Thionylchlorid pro Carboxylgruppe versetzt und diese Suspension oder Lösung auf 50—150° C, vorzugsweise 80—100° C, gebracht werden.

Nach Abdestillieren des überschüssigen Thionylchlorids und kurzzeitigem Anlegen von Vakuum bei Reaktionstemperatur erhält man einen Rückstand neben dem Katalysator der zu ≧99,9% aus aromatischem Carbonsäurechlorid besteht.

Es ist möglich, das Verfahren diskontinuierlich oder kontinuierlich durchzuführen.

## Beispiel

In einem 1l-Dreihalskolben mit Rührer, Thermometer und Rückflußkühler werden vorgelegt und aufgeheizt:

83 g Isophthalsäure
83 g Terephthalsäure
357 g Thionylchlorid
2 g Triphenylphosphin (1,2 Gew.-%, bezogen auf die Säuren).

Die blaßgelbe Suspension wird innerhalb von 30 Min. auf 60—70° C aufgeheizt, wobei zügige HCl- und $SO_2$-Entwicklung auftritt. Nach ca. 6 h ist die Gasentwicklung beendet und es liegt eine hellgelbe Lösung vor, aus der das überschüssige Thionylchlorid bei Normaldruck und danach im Vakuum entfernt wird.

Der Rückstand enthält neben dem Katalysator:

$\geqq$ 99,9% Säurechloride
$\leqq$ 0,05% COOH
$\leqq$ 0,01% S
$\leqq$ 0,05% Cl⁻

Mit diesem Säurechloridgemisch und Bisphenol A wurden in Gegenwart von o-Phenylphenol als Kettenabbrecher nach dem üblichen Grenzflächenpolykondensationsverfahren aromatische Polyester mit

a) $\eta$rel = 1,54 (2 Mol-% Kettenabbrecher) und
b) $\eta$rel = 1,27 (5 Mol-% Kettenabbrecher)

hergestellt.

## Patentansprüche

1. Einstufenverfahren zur Herstellung von reinen aromatischen Carbonsäurechloriden durch Umsetzung aromatischer Carbonsäuren mit Thionylchlorid, dadurch gekennzeichnet, daß als Katalysatoren tert. Phosphine oder deren Reaktionsprodukte mit Thionylchlorid und/oder den herzustellenden Säurechloriden verwendet werden.

2. Verfahren nach Anspruch 1, dadurch gekennzeichnet, daß der Katalysator eine Verbindung der Formel I

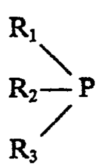

(I)

ist, in der $R_1$, $R_2$ und $R_3$ gleich oder verschieden und $C_1-C_8$-Alkyl, $C_6-C_{10}$-Aryl, $C_7-C_{20}$-Alkylaryl oder Arylalkyl sein können; bevorzugt sind $R_1$, $R_2$ und $R_3$ $C_6-C_{10}$-Arylreste, wie Phenyl oder mit $C_1-C_4$-Alkylresten substituiertes Phenyl.

## Claims

1. A single-stage process for the production of pure aromatic carboxylic acid chlorides by reacting aromatic carboxylic acids with thionyl chloride, characterised in that the catalysts used are tert. phosphines or the reaction products thereof with thionyl chloride and/or with the acid chlorides to be produced.

2. Process according to Claim 1, characterised in that the catalyst is a compound of the formula I

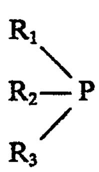

(I)

in which $R_1$, $R_2$ and $R_3$ can be identical or different and can be $C_1-C_8$-alkyl, $C_6-C_{10}$-aryl, $C_7-C_{20}$-alkylaryl or arylalkyl; $R_1$, $R_2$ and $R_3$ are preferably $C_6-C_{10}$-aryl radicals such as phenyl or phenyl substituted with $C_1-C_4$-alkyl radicals.

**Revendications**

1. Procédé en une étape pour la fabrication de chlorures d'acides carboxyliques aromatiques purs par réaction d'acides carboxyliques aromatiques avec le chlorure de thionyle, caractérisé en ce que l'on utilise comme catalyseurs des phosphines tertiaires ou leurs produits de réaction avec le chlorure de thionyle et/ou les chlorures d'acides à préparer.

2. Procédé selon la revendication I, caractérisé en ce que le catalyseur est un composé de formule

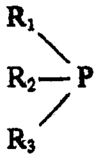

(I)

dans laquelle $R_1$, $R_2$ et $R_3$ sont identiques ou différents et peuvent être chacun un groupe alkyle en $C_1-C_8$, aryle en $C_6-C_{10}$, alkylaryle ou arylalkyle en $C_7-C_{20}$; de préférece $R_1$, $R_2$ et $R_3$ sont des restes aryles en $C_6-C_{10}$ tels que phényle ou phényle substitué par des restes alkyles en $C_1-C_4$.